# FASCICULE DE BREVET EUROPEEN

(11) **EP 0 595 694 B1**
(45) Date de publication et mention de la délivrance du brevet: **21.08.1996**
(21) Numéro de dépôt: 93402596.6
(22) Date de dépôt: 22.10.1993
(51) Int. Cl.: A61K 7/48

(54) **Composition cosmétique ou dermo-pharmaceutique contenant en association un lauroylméthionate d'un amino acide basique et au moins un polyphénol**
Kosmetische oder dermopharmazeutische Zusammensetzung, die eine Kombination aus einem Lauroylmethionat einer basischen Aminosäure und mindestens einem Polyphenol enthalten
Cosmetic or dermopharmaceutical composition containing in association a lauroylmethionate of a basic aminoacid and at least a polyphenol

(30) Priorité: 22.10.1992 FR 9212654
(43) Date de publication de la demande: 04.05.1994
(73) Titulaire: L'OREAL, F-75008 Paris (FR)
(72) Inventeur: N'Guyen, Quang Lan, F-92160 Antony (FR)
(74) Mandataire: Stalla-Bourdillon, Bernard

(56) Documents cités:
- EP-A- 0 275 005
- EP-A- 0 307 626
- EP-A- 0 353 161
- EP-A- 0 500 332
- FR-A- 1 603 799
- FR-A- 2 400 358

## Description

L'invention a pour objet une composition cosmétique ou dermo-pharmaceutique contenant en tant qu'antioxydant une association à action synergique d'un lauroylméthionate d'un amino acide basique et d'au moins un polyphénol.

La plupart des compositions cosmétiques ou dermo-pharmaceutiques sont constituées d'une phase grasse dont les produits huileux ont une certaine tendance à s'oxyder, même à température ambiante. Cette oxydation a pour conséquence d'en modifier profondément les propriétés, notamment olfactives, ce qui les rend inutilisables après une période de temps variable.

Afin de protéger les compositions vis-à-vis de ces phénomènes d'oxydation, il est de pratique courante d'incorporer des agents protecteurs qui jouent le rôle d'antioxydant.

Parmi les produits antioxydants que l'on utilise le plus couramment, on peut citer l'acide ascorbique qui agit notamment par absorption directe d'oxygène mais il présente cependant l'inconvénient d'être très peu soluble dans les corps gras, ce qui ne permet pas, dès lors, d'en assurer une bonne protection.

En vue de remédier aux inconvénients de l'acide ascorbique, divers systèmes antioxydants ont été dernièrement proposés, en particulier dans le brevet français n° 90 11384 (2.666.809), celui-ci étant constitué d'au moins un tocophérol ou un dérivé de tocophérol et d'au moins un polypeptide non thiolé.

De même, dans le brevet français n° 88 10295 (2.634.779), il a été suggéré l'emploi d'un système antioxydant constitué d'un tocophérol ou d'un mélange de tocophérols, ou de l'acide caféique ou l'un de ses dérivés, d'au moins un agent complexant et d'au moins un polypeptide non thiolé.

Ces systèmes antioxydants ont une action synergique particulièrement prononcée, mais ils sont néanmoins susceptibles de poser des problèmes de conservation lors de périodes de stockage prolongées.

Si les antioxydants sont particulièrement utiles pour la bonne conservation des corps gras des compositions cosmétiques ou dermo-pharmaceutiques, il convient également que ceux-ci, dans certaines applications, puissent permettre de lutter efficacement contre les effets nocifs des peroxydes, notamment les peroxydes organiques formés sous l'action des polluants atmosphériques et des ultra-violets.

Les cellules vivantes possèdent en effet divers moyens naturels de défense contre les peroxydes lipidiques en particulier la glutathion peroxydase épidermique, mais son efficacité détoxifiante est fortement diminuée sous l'influence d'une exposition aux ultra-violets.

Il est donc important de pouvoir disposer d'agents antioxydants capables d'inhiber la formation de radicaux libres qui sont une source de phénomènes d'oxydation pouvant provoquer des dommages cellulaires irréversibles.

Après diverses études ayant porté sur un grand nombre de substances, on a constaté de façon inattendue qu'il était possible à la fois d'obtenir une bonne conservation des compositions cosmétiques ou dermo-pharmaceutiques contenant des corps gras facilement oxydables et, par ailleurs, de protéger efficacement les lipides cutanés en utilisant un système antioxydant à action synergique constitué d'un lauroylméthionate d'un amino acide basique et d'au moins un polyphénol.

L'invention a donc pour objet une composition cosmétique ou dermo-pharmaceutique contenant dans un véhicule approprié, un système antioxydant à action synergique constitué par l'association d'un lauroylméthionate de lysine, d'histidine ou d'arginine et d'au moins un polyphénol choisi parmi :
a) un dérivé d'acide (2,5-dihydroxyphényl)carboxylique, un homologue ou un sel correspondant,
b) un ester ou amide de l'acide caféique,
c) un flavonoide ou un extrait à base de flavonoïdes,
d) un extrait de romarin contenant des diphénols,
   et leurs mélanges.

Parmi les lauroylméthionates, on préfère tout particulièrement utiliser selon l'invention le lauroylméthionate de L-lysine (PM = 477,7) vendu par la Société Givaudan Lavirotte.

Les dérivés d'acide (2,5-dihydroxyphényl)carboxylique, leurs homologues et leurs sels sont des composés connus qui ont été décrits notamment dans les brevets français 78.24174 (2.400.358) et 78.24175 (2.400.359).

Ces dérivés d'acide (2,5-dihydroxyphényl)carboxylique peuvent être représentés par la formule générale suivante : 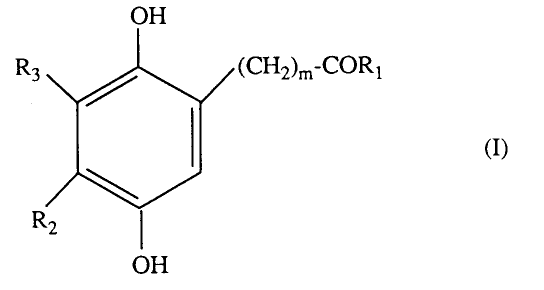
dans laquelle :
R₁ représente OR₄, OH ou 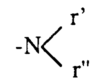
R₄ représente un radical alkyle en C₁-C₂₀, linéaire ou ramifié, un radical alcényle en C₂-C₂₀, linéaire ou ramifié, un radical alkyle en C₁-C₂₀ substitué par un ou plusieurs groupements hydroxy ou alcoxy,
r' et r'', identiques ou différents, représentent un atome d'hydrogène, un radical alkyle en C₁-C₂₀, un radical hydroxyalkyle en C₂-C₆ ou un radical polyhydroxyalkyle en C₃-C₆ ou r' et r'', pris ensemble, forment avec l'atome d'azote un hétérocycle,
m est 1 ou 2,
(i) lorsque m est 1, l'un au moins des radicaux R₂ et R₃ représente un radical alkyle en C₁-C₄ linéaire ou ramifié, l'autre représentant éventuellement un atome d'hydrogène,
(ii) lorsque m est 2, R₂ et R₃, identiques ou différents, représentent un atome d'hydrogène ou un radical alkyle linéaire ou ramifié en C₁-C₄.

Parmi les composés particulièrement préférés de formule générale (I), on peut notamment citer le 2,5-dihydroxy 4-méthylphényl acétate de méthyle.

Parmi les esters de l'acide caféique, on peut mentionner notamment les composés de formule (II) : 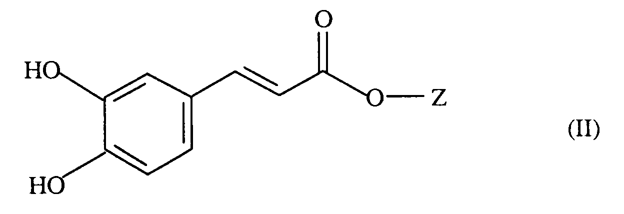
dans laquelle Z représente un alkyle C₁-C₈, par exemple méthyle, ou le reste d'un phytol.

Parmi les amides de l'acide caféique, on peut citer notamment les composés de formule (III) : 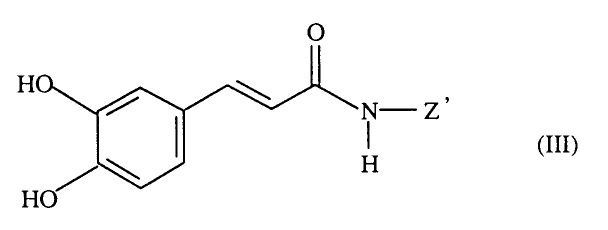
dans laquelle Z' représente un alkyle en C₁-C₈, en particulier en C₆-C₈.

Les composés de formule (II) ou (III) sont connus ou peuvent être préparés selon les méthodes connues.

Parmi les flavonoïdes, on peut mentionner ceux répondant aux deux formules générales suivantes : 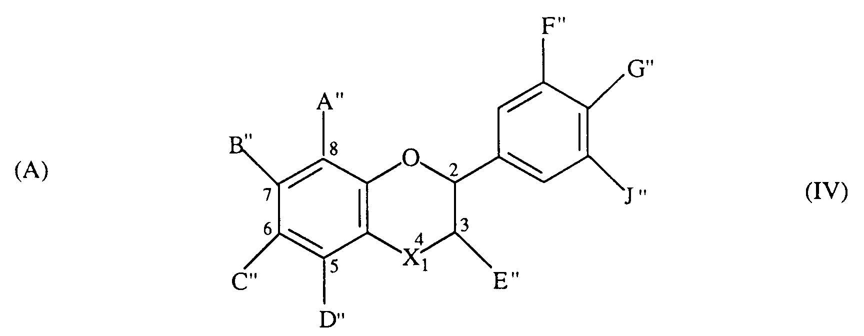
dans laquelle :
A'', B'', C'', et D'', indépendamment les uns des autres, représentent H ou OH ;
E'' représente H, OH ou OX', où X' représente : 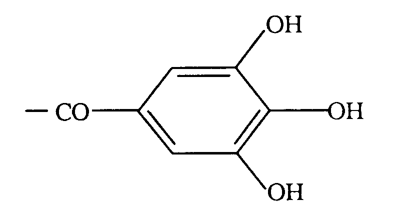
F'', G'', J'' représentent, indépendamment les uns des autres, H ou OH ; et X₁ représente - CH₂-, -CO- ou -CHOH-,
sous réserve qu'au moins deux des radicaux A'' à G'' ou J'' représentent OH ou que le radical E'' représente OX', et 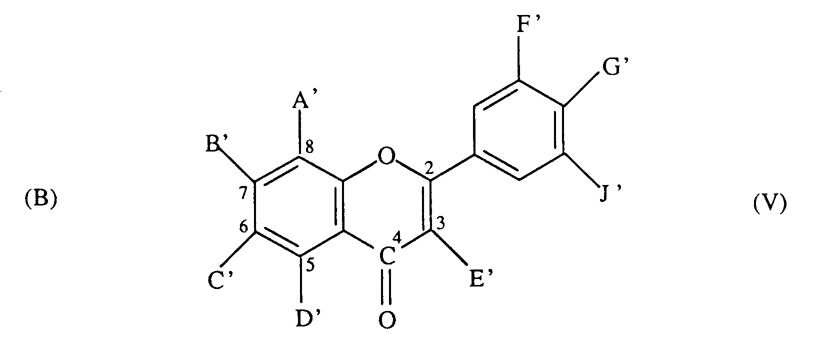 dans laquelle :
A', C' et D', indépendamment les uns des autres, représentent H, OH ou OCH₃ ;
E' représente H, OH ou OR', où R' représente le reste d'un sucre de formule R'OH ;
B', F', G' et J', indépendamment les uns des autres, représentent H, OH, OCH₃ ou -OCH₂-CH₂-OH,
sous réserve qu'au moins deux des radicaux A' à G' ou J' représentent OH.
Parmi les sucres R'OH, on peut citer le rutinose.

Les composés de formule (IV) et (V) sont connus. Ils peuvent être obtenus notamment selon les procédés décrits dans "The Flavonoids" Harborne J.B., Mabry T.J., Helga Mabry, 1975, pages 1 à 45.

Parmi les flavonoïdes utilisables selon l'invention, on citera notamment la taxifoline, la catéchine, l'épicatéchine, l'eriodictyol, la naringénine, la rutine, la troxérutine, la chrysine, la tangérétine, la lutéoline, l'épigallocatéchine et le gallate de l'épigallocatéchine, la quercétine, la fisétine, le kaëmpférol, la galangine, la gallocatéchine, le gallate d'épicatéchine.

De tels composés se trouvent notamment dans des extraits de thé vert vendus sous la dénomination "Sunphenon®" par la Société Nikko.

L'extrait de romarin utilisable selon l'invention, est essentiellement caractérisé par la présence de l'acide carnosique et du carnosol et peut être obtenu soit par extraction suivie d'une distillation (Chang et al. JOSC, Vol.61, n°6, Juin 1984), soit par une extraction par un solvant polaire tel que l'éthanol précédée par une extraction à l'aide d'un solvant non polaire tel que l'hexane pour éliminer les substances odorantes, comme décrit dans la demande de brevet EP-307 626.

L'acide carnosique et le carnosol répondent aux formules suivantes :

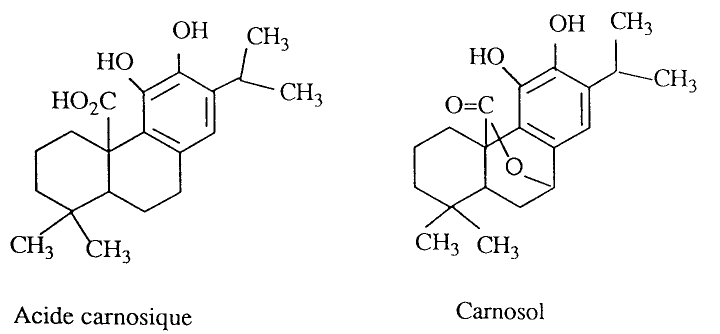

Dans les compositions cosmétiques ou dermo-pharmaceutiques selon l'invention, le lauroylméthionate de lysine, d'histidine ou d'arginine est généralement présent à une concentration comprise entre 0,02 % et 5 % en poids par rapport au poids total de la composition.

Le polyphénol tel que défini ci-dessus est présent en une proportion comprise entre 0,005 et 5 % en poids par rapport au poids total de la composition. Le rapport en poids entre le lauroylméthionate d'amino acide basique et le polyphénol est généralement compris entre 20/80 et 90/10.

Lorsque le polyphénol est un dérivé d'acide (2,5-dihydroxyphényl)carboxylique tel que représenté par la formule générale (I), celui-ci est de préférence présent à une concentration comprise entre 0,05 et 1 %.

Si celui-ci est un ester ou amide de l'acide caféique, la concentration est de préférence comprise entre 0,05 et 2 % en poids par rapport au poids total de la composition.

Enfin, lorsque l'on utilise un flavonoïde, ou un extrait contenant des flavonoïdes, la concentration est généralement comprise entre 0,1 et 5 % en poids par rapport au poids total de la composition.

De même lorsque l'on utilise un extrait de romarin contenant de l'acide carnosique et du carnosol la concentration est généralement comprise entre 0,1 et 5% en poids par rapport au poids total de la composition.

Les compositions selon l'invention peuvent être de nature très variée mais sont plus particulièrement celles contenant des corps gras sensibles à l'oxydation en une proportion variable, de préférence comprise entre 0,1 et 99 % en poids.

Les corps gras présents dans les compositions selon l'invention sont par exemple des corps gras d'origine animale tels que la cétine (blanc de baleine), la cire d'abeilles, la lanoline, le perhydrosqualène, l'huile de tortue, etc; des corps gras végétaux sous forme d'huiles, de graisses ou de cires telles que l'huile d'amande douce, l'huile d'avocat, l'huile d'olive, l'huile de sésame, l'huile de macadamia; les huiles de coprah ou de palmiste éventuellement hydrogénées, le beurre de cacao, la cire de carnauba, la cire de montana; ainsi que des huiles synthétiques constituées par des esters et/ou éthers de glycérol ou de glycol tels que par exemple ceux décrits dans les brevets français n° 75.24656 (2.281.916), 75.24657 (2.281.743) et 75.24658 (2.281.744).

En plus des corps gras plus ou moins oxydables, les compositions cosmétiques ou dermo-pharmaceutiques selon l'invention peuvent également contenir des produits sensibles à l'oxydation tels que par exemple de la vitamine F ou du β-carotène.

Les compositions cosmétiques ou dermo-pharmaceutiques selon l'invention se présentent notamment sous forme de solutions huileuses, d'émulsions eau-dans-l'huile ou huile-dans-l'eau, de produits solides éventuellement anhydres, de lotions ou de microdispersions, de dispersions vésiculaires, les lipides constitutifs des vésicules pouvant être du type ionique ou non-ionique ou bien un mélange de ceux-ci. Les compositions selon l'invention peuvent également se présenter sous forme de laits pour les soins de la peau, de crèmes (crèmes pour le visage, pour les mains, pour le corps, crèmes anti-solaires, crèmes démaquillantes, crèmes fonds de teint), de fonds de teint fluides, de laits démaquillants, de laits anti-solaires, d'huiles pour le bain, de rouges à lèvres, de fards à paupières, de sticks déodorants, etc.

Pour l'application par voie topique, les compositions cosmétiques ou dermo-pharmaceutiques selon l'invention comprennent les véhicules et ingrédients nécessaires pour permettre de présenter la composition par exemple sous la forme d'onguents, de crèmes, de laits, de pommades et de solutions huileuses.

Selon une forme de réalisation préférée, les compositions cosmétiques ou dermo-pharmaceutiques se présentent sous une forme destinée à être appliquée par voie topique, en particulier de crème destinée à la protection de l'oxydation des lipides de la peau.

Les compositions cosmétiques et dermo-pharmaceutiques selon l'invention peuvent en outre contenir divers additifs ou ingrédients usuels, tels que des agents tensioactifs, des colorants, des parfums, des produits astringents, des produits absorbant l'ultra-violet, des solvants organiques.

Ces compositions cosmétiques ou dermo-pharmaceutiques sont obtenues selon les méthodes conventionnelles.

On va maintenant donner à titre d'illustration plusieurs exemples de compositions cosmétiques et dermo-pharmaceutiques selon l'invention.

| **EXEMPLE 1 : *Crème hydratante*** | |
|---|---|
| | % |
| - Lauroylméthionate de lysine | 1,15 |
| - 2,5-dihydroxy 4-méthylphényl acétate de méthyle | 1,0 |
| - Lanolate de Mg | 3,0 |
| - Alcool de lanoline | 5,0 |
| - Huile de vaseline | 27,0 |
| - Vaseline | 15,0 |
| - Parahydroxybenzoate de méthyle | 0,2 |
| - Parahydroxybenzoate de propyle | 0,1 |
| - Eau déminéralisée q.s.p. | 100 |

| **EXEMPLE 2 : *Crème protectrice de jour*** | |
|---|---|
| | % |
| - Lauroylméthionate de lysine | 1,3 |
| - Quercétine | 0,5 |
| - Extrait de romarin | 1,0 |
| - Stéarate de glycérol auto-émulsionnable | 3,0 |
| - Alcool cétylique | 0,5 |
| - Alcool stéarylique | 0,5 |
| - Huile de vaseline | 12,0 |
| - Huile de sésame | 10,0 |
| - Acide stéarique | 3,0 |
| - Parahydroxybenzoate de méthyle | 0,2 |
| - Parahydroxybenzoate de propyle | 0,1 |
| - Parfum | 0,3 |
| - Eau déminéralisée q.s.p. | 100 |

| **EXEMPLE 3 : *Crème protectrice hydratante*** | |
|---|---|
| | % |
| - Lauroylméthionate de lysine | 2,3 |
| - Flavonoïdes Extraits de thé vert ("Sunphenon®" de la Société Nikko) | 1,0 |
| - Stéarate de glycérol | 3,0 |
| - Alcool stéarylique | 0,5 |
| - Acide stéarique | 2,0 |
| - Perhydrosqualène | 12,0 |
| - Huile de silicone volatile | 5,0 |
| - Parahydroxybenzoate de méthyle | 0,2 |
| - Parahydroxybenzoate de propyle | 0,1 |
| - Parfum | 0,3 |
| - Eau déminéralisée q.s.p. | 100 |

| **EXEMPLE 4 : *Crème protectrice pour les mains*** | |
|---|---|
| | % |
| - Lauroylméthionate de lysine | 0,345 |
| - Caféate de méthyle | 0,4 |
| - Rutine | 0,5 |
| - Monostéarate de sorbitan polyoxyéthyléné à 20 moles d'oxyde d'éthylène | 2,0 |
| - Alcool cétylique | 1,0 |
| - Myristate d'isopropyle | 3,0 |
| - Huile de vaseline | 7,0 |
| - Huile de silicone volatile | 7,0 |
| - Parahydroxybenzoate de méthyle | 0,2 |
| - Parahydroxybenzoate de propyle | 0,1 |
| - Eau déminéralisée q.s.p. | 100 |

| **EXEMPLE 5 : *Dispersion vésiculaire*** | |
|---|---|
| | % |
| - Lauroylméthionate de lysine | 0,23 |
| - 2,5-dihydroxy 4-méthylphényl acétate de méthyle | 0,05 |
| - Lécithine de soja hydrogénée | 1,8 |
| - Cholestérol | 0,9 |
| - Lipacide palmitoyl collagénique | 0,3 |
| - Glycérine | 0,3 |
| - Huile de macadamia | 15,0 |
| - Huile de silicone volatile | 10,0 |
| - Polymère carboxyvinylique vendu sous la dénomination de "Carbopol® 940" par la Société Goodrich | 0,6 |
| - Parahydroxybenzoate de méthyle | 0,2 |
| - Triéthanolamine q.s. pH = 6 | |
| - Eau déminéralisée q.s.p. | 100 |

## Revendications

1. Composition cosmétique ou dermo-pharmaceutique, caractérisée par le fait qu'elle contient dans un véhicule approprié, un système antioxydant à action synergique constitué par l'association d'un lauroylméthionate de lysine, d'histidine ou d'arginine, et d'au moins un polyphénol choisi parmi :
a) un dérivé d'acide (2,5-dihydroxyphényl)carboxylique, un homologue ou un sel correspondant,
b) un ester ou amide de l'acide caféique,
c) un flavonoide ou un extrait à base de flavonoïdes,
d) un extrait de romarin contenant des diphénols,
et leurs mélanges

2. Composition selon la revendication 1, caractérisée par le fait que le système antioxydant contient du lauroylméthionate de lysine.

3. Composition selon la revendication 1, caractérisée par le fait que le dérivé d'acide (2,5-dihydroxyphényl)carboxylique correspond à la formule générale suivante : 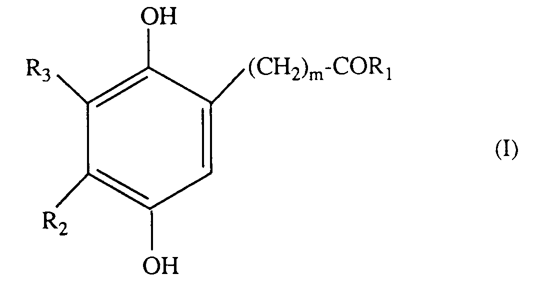 dans laquelle :
R₁ représente OR₄, OH ou 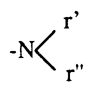 R₄ représente un radical alkyle en C₁-C₂₀, linéaire ou ramifié, un radical alcényle en C₂-C₂₀, linéaire ou ramifié, un radical alkyle en C₁-C₂₀ substitué par un ou plusieurs groupements hydroxy ou alcoxy,
r' et r'', identiques ou différents, représentent un atome d'hydrogène, un radical alkyle en C₁-C₂₀, un radical hydroxyalkyle en C₂-C₆ ou un radical polyhydroxyalkyle en C₃-C₆ ou r' et r'', pris ensemble, forment avec l'atome d'azote un hétérocycle,
m est 1 ou 2,
(i) lorsque m est 1, l'un au moins des radicaux R₂ et R₃ représente un radical alkyle en C₁-C₄ linéaire ou ramifié, l'autre représentant éventuellement un atome d'hydrogène,
(ii) lorsque m est 2, R₂ et R₃, identiques ou différents, représentent un atome d'hydrogène ou un radical alkyle linéaire ou ramifié en C₁-C₄.

4. Composition selon l'une quelconque des revendications 1 à 3, caractérisée par le fait que le dérivé d'acide (2,5-dihydroxyphényl)carboxylique est le 2,5-dihydroxy 4-méthylphényl acétate de méthyle.

5. Composition selon la revendication 1, caractérisée par le fait que l'ester de l'acide caféique répond à la formule : 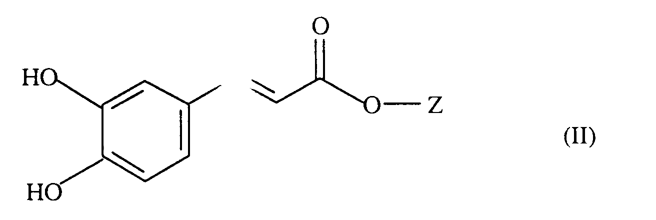 dans laquelle Z représente un alkyle C₁-C₈, par exemple méthyle, ou le reste d'un phytol.

6. Composition selon la revendication 1, caractérisée par le fait que l'amide de l'acide caféique répond à la formule : 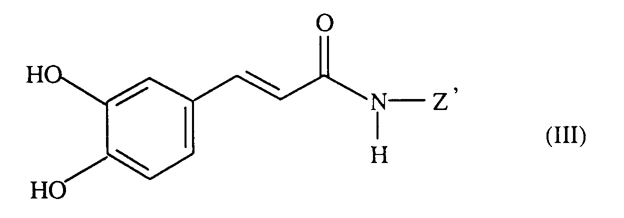 dans laquelle Z' représente un alkyle en C₁-C₈, en particulier en C₆-C₈.

7. Composition selon la revendication 1, caractérisée par le fait que le flavonoïde répond à l'une des deux formules suivantes : 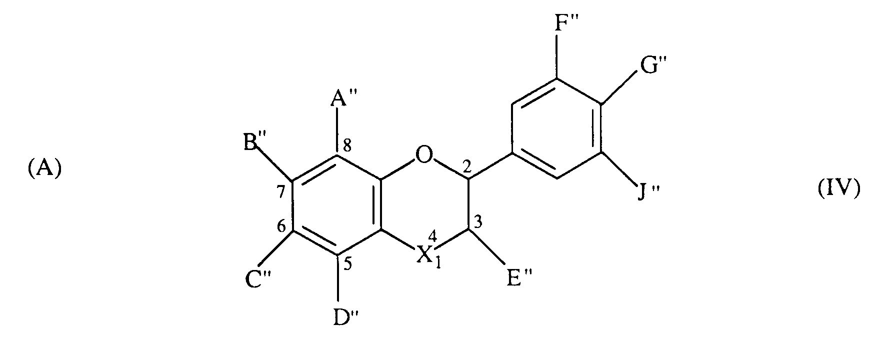 dans laquelle :
A'', B'', C'', et D'', indépendamment les uns des autres, représentent H ou OH ;
E'' représente H, OH ou OX', où X' représente : 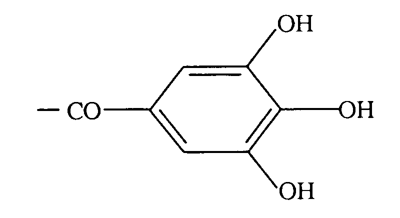 F'', G'', J'' représentent, indépendamment les uns des autres, H ou OH ; et X₁ représente -CH₂-, -CO- ou -CHOH-,
sous réserve qu'au moins deux des radicaux A'' à G'' ou J'' représentent OH ou que le radical E'' représente OX', et 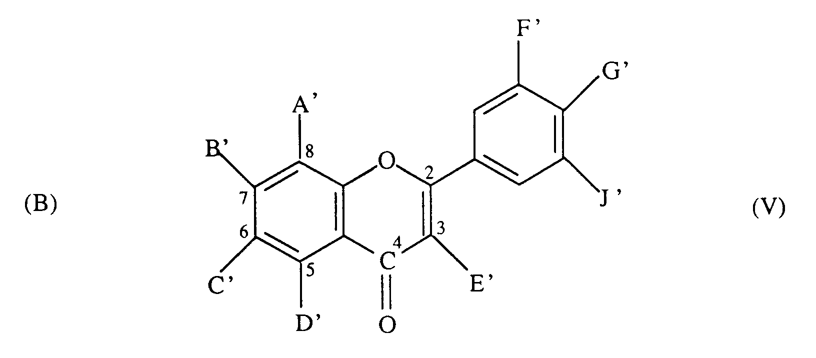 dans laquelle :
A', C' et D', indépendamment les uns des autres, représentent H, OH ou OCH₃;
E' représente H, OH ou OR', où R' représente le reste d'un sucre de formule R'OH ;
B', F', G' et J', indépendamment les uns des autres, représentent H, OH, OCH₃ ou -OCH₂-CH₂-OH,
sous réserve qu'au moins deux des radicaux A' à G' ou J' représentent OH.

8. Composition selon l'une quelconque des revendications précédentes, caractérisée par le fait que le lauroylméthionate de lysine, d'histidine ou d'arginine est présent à une concentration comprise entre 0,02 % et 5 % par rapport au poids total de la composition.

9. Composition selon l'une quelconque des revendications précédentes, caractérisée par le fait que le polyphénol est présent à une concentration comprise entre 0,005 et 5 % en poids par rapport au poids total de la composition.

10. Composition selon l'une quelconque des revendications 1 à 4, caractérisée par le fait que le dérivé d'acide (2,5-dihydroxyphényl) carboxylique est présent à une concentration comprise entre 0,05 et 1 % en poids par rapport au poids total de la composition.

11. Composition selon l'une quelconque des revendications 1, 2, 5 et 6, caractérisée par le fait que l'ester et/ou amide de l'acide caféique est présent à une concentration comprise entre 0,05 et 2 % en poids par rapport au poids total de la composition.

12. Composition selon l'une quelconque des revendications 1, 2 et 7, caractérisée par le fait que le flavonoïde, ou un extrait à base de flavonoïdes est présent à une concentration comprise entre 0,1 et 5 % en poids par rapport au poids total de la composition.

13. Composition selon les revendications 1 et 2, caractérisée par le fait que l'extrait de romarin contient de l'acide carnosique et du carnosol et est présent à une concentration comprise entre 0,1 et 5% en poids par rapport au poids total de la composition.

14. Composition selon l'une quelconque des revendications précédentes, caractérisée par le fait qu'elle contient au moins un corps gras sensible à l'oxydation en une proportion comprise entre 1 et 99 % en poids par rapport au poids total de la composition.

15. Composition selon l'une quelconque des revendications précédentes, caractérisée par le fait qu'elle contient au moins un produit sensible à l'oxydation choisi parmi la vitamine F ou le β-carotène.

16. Composition selon l'une quelconque des revendications précédentes, caractérisée par le fait qu'elle contient en outre divers additifs choisis parmi les agents tensioactifs, les colorants, les parfums, les produits astringents, les produits absorbant l'ultra-violet, et les solvants organiques.

## Claims

1. Cosmetic or dermopharmaceutical composition, characterized in that it contains, in a suitable vehicle, an antioxidizing system possessing a synergic effect consisting of the combination of a lauroylmethionate of lysine, of histidine or of arginine, and of at least one polyphenol chosen from:
a) a derivative of (2,5-dihydroxyphenyl)carboxylic acid, a homologue or a corresponding salt,
b) an ester or amide of caffeic acid,
c) a flavonoid, or a flavonoid-based extract
d) a rosemary extract containing diphenols, and their mixtures.

2. Composition according to Claim 1, characterized in that the antioxidizing system contains lysine lauroylmethionate.

3. Composition according to Claim 1, characterized in that the derivative of (2,5-dihydroxyphenyl)carboxylic acid corresponds to the following general formula: 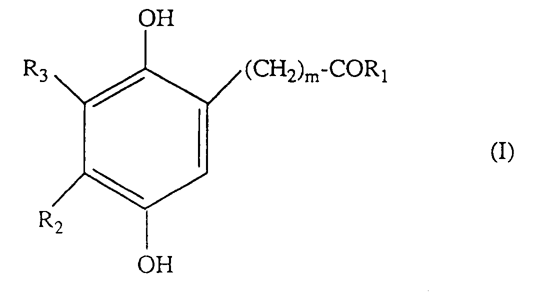 in which:
R₁ represents OR₄, OH or 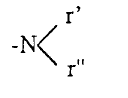 R₄ represents a linear or branched C₁-C₂₀ alkyl radical, a linear or branched C₂-C₂₀ alkenyl radical or a C₁-C₂₀ alkyl radical substituted by one or a number of hydroxyl or alkoxy groups,
r' and r'', which are identical or different, represent a hydrogen atom, a C₁-C₂₀ alkyl radical, a C₂-C₆ hydroxyalkyl radical or a C₃-C₆ polyhydroxyalkyl radical or r' and r'', taken together, form with the nitrogen atom a heterocycle,
m is 1 or 2,
(i) when m is 1, at least one of the R₂ and R₃ radicals represents a linear or branched C₁-C₄ alkyl radical, the other optionally representing a hydrogen atom,
(ii) when m is 2, R₂ and R₃, which are identical or different, represent a hydrogen atom or a linear or branched C₁-C₄ alkyl radical.

4. Composition according to any one of Claims 1 to 3, characterized in that the derivative of (2,5-dihydroxyphenyl)carboxylic acid is methyl 2,5-dihydroxy-4-methylphenylacetate.

5. Composition according to Claim 1, characterized in that the ester of caffeic acid corresponds to the formula: 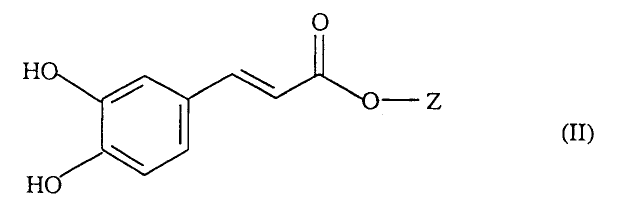 in which Z represents a C₁-C₈ alkyl, for example methyl, or the residue of a phytol.

6. Composition according to Claim 1, characterized in that the amide of caffeic acid corresponds to the formula: 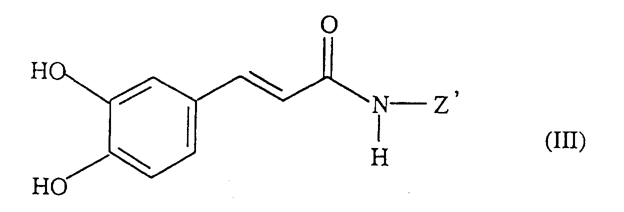 in which Z' represents a C₁-C₈, in particular C₆-C₈, alkyl.

7. Composition according to Claim 1, characterized in that the flavonoid corresponds to one of the following two formulae: 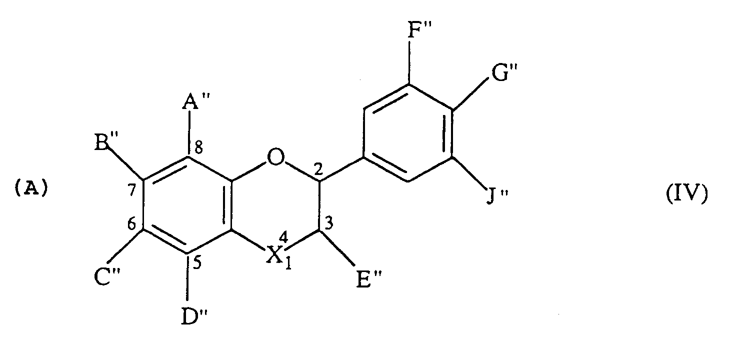 in which:
A'', B'', C'' and D'', independently of one another, represent H or OH;
E'' represents H, OH or OX', where X' represents: 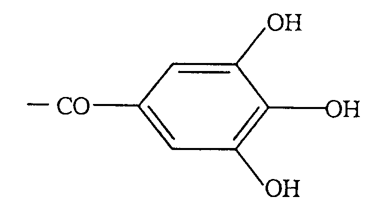 F'', G'', J'' represent, independently of one another, H or OH; and X₁ represents -CH₂-, -CO- or -CHOH-,
with the proviso that at least two of the radicals A'' to G'' or J'' represent OH or that the E'' radical represents OX', and 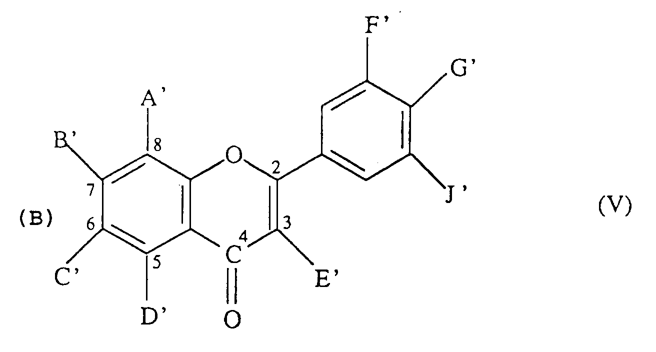 in which:
A', C' and D', independently of one another, represent H, OH or OCH₃;
E' represents H, OH or OR', where R' represents the residue of a sugar of formula R'OH;
B', F', G' and J', independently of one another, represent H, OH, OCH₃ or -OCH₂-CH₂-OH,
with the proviso that at least two of the radicals A' to G' or J' represent OH.

8. Composition according to any one of the preceding claims, characterized in that the lauroylmethionate of lysine, of histidine or of arginine is present at a concentration between 0.02 % and 5 % with respect to the total weight of the composition.

9. Composition according to any one of the preceding claims, characterized in that the polyphenol is present at a concentration between 0.005 and 5 % by weight with respect to the total weight of the composition.

10. Composition according to any one of Claims 1 to 4, characterized in that the derivative of (2,5-dihydroxyphenyl)carboxylic acid is present at a concentration between 0.05 and 1 % by weight with respect to the total weight of the composition.

11. Composition according to any one of Claims 1, 2, 5 and 6, characterized in that the ester and/or amide of caffeic acid is present at a concentration between 0.05 and 2 % by weight with respect to the total weight of the composition.

12. Composition according to any one of Claims 1, 2 and 7, characterized in that the flavonoid, or a flavonoid-based extract, is present at a concentration between 0.1 and 5 % by weight with respect to the total weight of the composition.

13. Composition according to Claims 1 and 2, characterized in that the rosemary extract contains carnosic acid and carnosol and is present at a concentration between 0.1 and 5 % by weight with respect to the total weight of the composition.

14. Composition according to any one of the preceding claims, characterized in that it contains at least one oxidation-sensitive fat in a proportion between 1 and 99 % by weight with respect to the total weight of the composition.

15. Composition according to any one of the preceding claims, characterized in that it contains at least one oxidation-sensitive product chosen from vitamin F or β-carotene.

16. Composition according to any one of the preceding claims, characterized in that it additionally contains various additives chosen from surface-active agents, dyes, fragrances, astringent products, products which absorb ultraviolet radiation, and organic solvents.

## Patentansprüche

1. Kosmetische oder dermopharmazeutische Zusammensetzung, dadurch gekennzeichnet, daß sie in einem geeigneten Träger ein oxidationshemmendes System mit synergetischer Wirkung enthält, bestehend aus einer Kombination aus einem Lysin-, Histidin- oder Argininlauroylmethionat und zumindest einem Polyphenol enthält, ausgewählt aus:
a) einem Derivat der (2,5-Dihydroxyphenyl)-Carbonsäure, einem Homolog oder einem entsprechenden Salz,
b) einem Kaffeesäureester oder -amid,
c) einem Flavonoid oder einem Extrakt auf Flavonoidbasis
d) einem Rosmarinextrakt, das Diphenole enthält,
und deren Gemischen.

2. Zusammensetzung nach Anspruch 1, dadurch gekennzeichnet, daß das oxidationshemmende System Lysinlauroylmethionat enthält.

3. Zusammensetzung nach Anspruch 1, dadurch gekennzeichnet, daß das Derivat der (2,5-Dihydroxyphenyl)-carbonsäure folgender allgemeinen Formel entspricht: 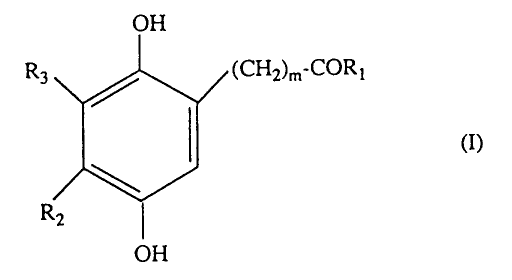 in der
R₁ OR₄, OH oder 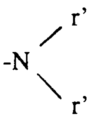 darstellt,
R₄ ein lineares oder verzweigtes C₁-C₂₀-Alkylradikal, ein lineares oder verzweigtes C₂-C₂₀ -Alkenylradikal, ein C₁-C₂₀-Alkylradikal, substituiert durch eine oder mehrere Hydroxy- oder Alkoxy-Gruppen, darstellt,
r' und r'' identisch oder verschieden sind und ein Wasserstoffatom, ein C₁-C₂₀-Alkylradikal, ein C₂-C₆-Hydroxyalkylradikal oder ein C₃-C₆-Polyhydroxyalkylradikal bedeuten, oder r' und r'' zusammengenommen mit dem Stickstoffatom eine heterocyclische Verbindung bilden, und
m = 1 oder 2 ist,
(i) wenn m = 1 ist, zumindest eines der Radikale R₂ und R₃ ein lineares oder verzweigtes C₁- C₄- Alkylradikal darstellen, wobei das andere gegebenenfalls ein Wassetstoffatom darstellt,
(ii) wenn m = 2 ist, R₂ und R₃, identisch oder verschieden, ein Wasserstoffatom oder ein lineares oder verzweigtes C₁-C₄-Alkylradikal darstellen.

4. Zusammensetzung nach einem der Ansprüche 1 bis 3, dadurch gekennzeichnet, daß das Derivat der (2,5-Dihydroxyphenyl)-carbonsäure Methyl-2,5-dihydroxy-4-methylphenyl-acetat ist.

5. Zusammensetzung nach Anspruch 1, dadurch gekennzeichnet, daß der Kaffeesäureester folgender Formel entspricht: 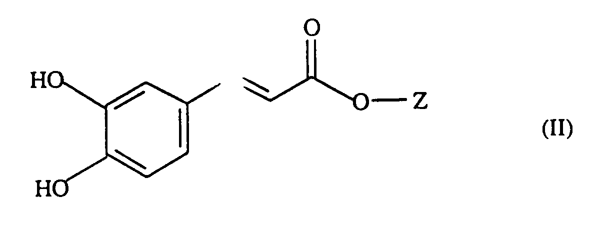 in der Z ein C₁- C₈- Alkyl z. B. Methyl oder einen Phytolrest darstellt.

6. Zusammensetzung nach Anspruch 1, dadurch gekennzeichnet, daß das Kaffeesäureamid folgender Formel entspricht: 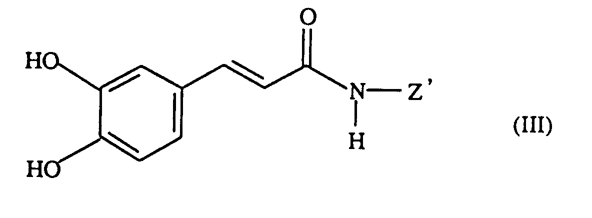 in der Z' ein C₁-C₈-Alkyl, insbesondere C₆-C₈ darstellt.

7. Zusammensetzung nach Anspruch 1, dadurch gekennzeichnet, daß das Flavonoid einer der beiden folgenden Formeln entspricht: 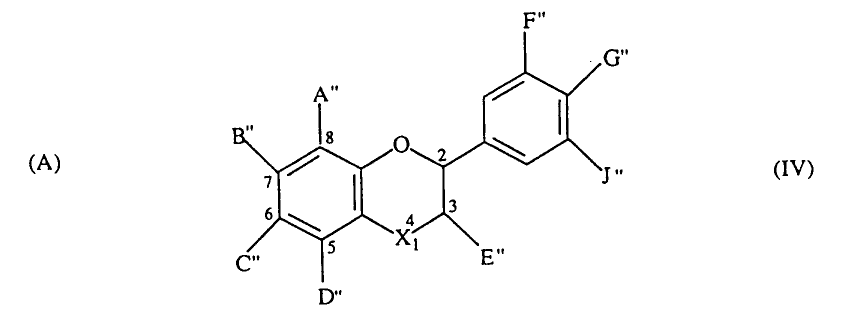 in der A'', B'', C'' und D'' unabhängig voneinander H oder OH darstellen,
E'' H, OH oder OX' darstellt, in der X' 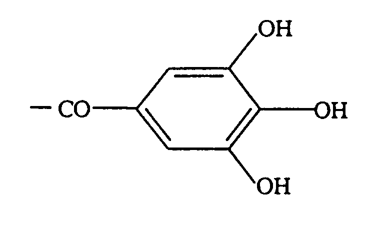 darstellt,
F'', G'', J'' unabhängig voneinander H oder OH darstellen und
X₁ -CH₂-, -CO- oder -CHOH- darstellt,
unter der Bedingung, daß mindestens zwei der Radikale A'' bis G'' oder J'' OH darstellen oder daß das Radikal E'' OX' darstellt, und 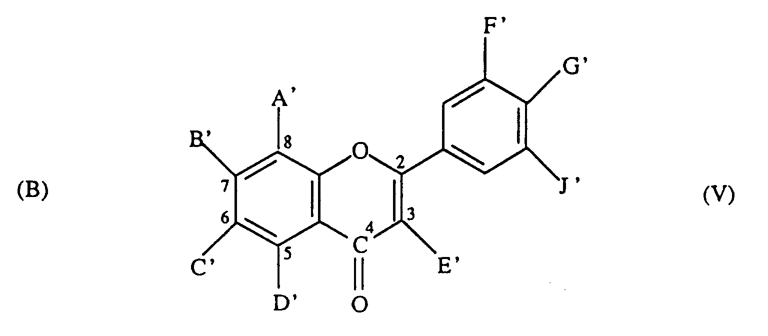 in der
A', C' und D' unabhängig voneinander H, OH oder OCH₃ darstellen,
E' H, OH oder OR' darstellt, wobei R' einen Zuckerrest der Formel R'OH darstellt, und
B', F', G' und J' unabhängig voneinander H, OH, OCH₃ oder -OCH₂-CH₂-OH darstellen,
unter der Bedingung, daß zumindest zwei der Radikale A' bis G' oder J' OH darstellen.

8. Zusammensetzung nach einem der vorhergehenden Ansprüche, dadurch gekennzeichnet, daß das Lysin-, Histidin- oder Argininlauroylmethionat in einer Konzentration von 0,02 bis 5 Gew.-%, bezogen auf das Gesamtgewicht der Zusammensetzung, vorhanden ist.

9. Zusammensetzung nach einem der vorhergehenden Ansprüche, dadurch gekennzeichnet, daß das Polyphenol in einer Konzentration von 0,005 und 5 Gew.-%, bezogen auf das Gesamtgewicht der Zusammensetzung, vorhanden ist.

10. Zusammensetzung nach einem der Ansprüche 1 bis 4, dadurch gekennzeichnet, daß das Derivat der (2,5-Dihydroxyphenyl)-carbonsäure in einer Konzentration von 0,05 und 1 Gew.-%, bezogen auf das Gesamtgewicht der Zusammensetzung, vorhanden ist.

11. Zusammensetzung nach einem der Ansprüche 1, 2, 5 und 6, dadurch gekennzeichnet, daß Koffeinsäureester und/oder -amid in einer Konzentration von 0,05 und 2 Gew.-%, bezogen auf das Gesamtgewicht der Zusammensetzung vorhanden ist.

12. Zusammensetzung nach einem der Ansprüche 1, 2 und 7, dadurch gekennzeichnet, daß das Flavonoid oder ein Extrakt auf Flavonoidbasis in einer Konzentration von 0,1 und 5 Gew.-%, bezogen auf das Gesamtgewicht der Zusammensetzung, vorhanden ist.

13. Zusammensetzung nach Anspruch 1 und 2, dadurch gekennzeichnet, daß der Rosmarinextrakt Carnosinsäure oder Carnosin enthält und in einer Konzentration von 0,1 und 5 Gew.-%, bezogen auf das Gesamtgewicht der Zusammensetzung, vorhanden ist.

14. Zusammensetzung nach einem der vorhergehenden Ansprüche, dadurch gekennzeichnet, daß sie zumindest einen oxidationsempfindlichen Fettkörper in einer Menge von 1 und 99 Gew.-%, bezogen auf das Gesamtgewicht der Zusammensetzung, enthält.

15. Zusammensetzung nach einem der vorhergehenden Ansprüche, dadurch gekennzeichnet, daß sie zumindest eine oxidationsempfindliche Substanz, ausgewählt aus Vitamin F oder β-Carotin, enthält.

16. Zusammensetzung nach einem der vorhergehenden Ansprüche, dadurch gekennzeichnet, daß sie außerdem verschiedene Zusätze enthält, die aus grenzflächenaktiven Stoffen, Farbstoffen, Duftstoffen, adstringierenden Stoffen, UV-Licht absorbierenden Mitteln und organischen Lösungsmitteln ausgewählt sind.
